(19) Europäisches Patentamt
European Patent Office
Office européen des brevets

(11) Veröffentlichungsnummer: **0 002 445 A1**

(12) **EUROPÄISCHE PATENTANMELDUNG**

(21) Anmeldenummer: **78101271.1**

(22) Anmeldetag: **02.11.78**

(51) Int. Cl.[2]: **C 07 D 233/76**
C 08 G 18/78, C 09 D 3/49
C 09 J 3/16

(30) Priorität: **12.11.77 DE 2750772**

(43) Veröffentlichungstag der Anmeldung:
**27.06.79 Patentblatt 79/13**

(84) Benannte Vertragsstaaten:
**DE FR GB**

(71) Anmelder: **Bayer Aktiengesellschaft**
**Zentralbereich Patente,Marken und Lizenzen Bayerwerk**
**D-5090 Leverkusen 1(DE)**

(72) Erfinder: **Lewalter, Jürgen, Dr.**
**Bergstrasse 88**
**D-5068 Odenthal(DE)**

(72) Erfinder: **Merten, Rudolf, Dr.**
**Berta-von-Suttner-Strasse 55**
**D-5090 Leverkusen 1(DE)**

(72) Erfinder: **Zecher, Wilfried, Dr.**
**Treptower Strasse 6**
**D-5090 Leverkusen 1(DE)**

(72) Erfinder: **Dünwald, Willi, Dr.**
**Geschwister-Scholl-Strasse 16**
**D-5090 Leverkusen 1(DE)**

(54) **Einen Hydantoin- oder Thiohydantoin-Ring enthaltende Polyiso(thio) cyanate, ihre Herstellung und Verwendung.**

(57) (Thio)hydantoingruppen-haltige Polyiso(thio)cyanate der allgemeinen Formel (I)

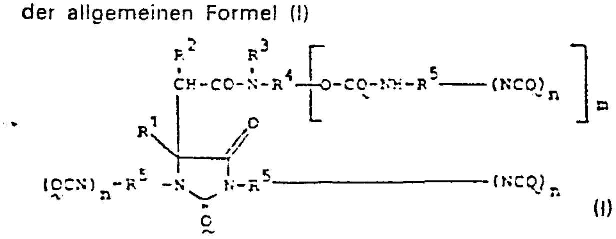

(I)

in der

$R^1$ und $R^2$ gleich oder verschieden, gegebenenfalls substituierte aliphatische, aliphatisch-aromatische oder aromatische Reste, Wasserstoff oder Halogen,

$R^4$ und $R^5$ gleich oder verschieden, einen mindestens zweiwertigen, gegebenenfalls substituierten aliphatischen, aliphatisch-aromatischen oder aromatischen Rest,

$R^3$ Wasserstoff, einen einwertigen, gegebenenfalls substituierten aliphatischen, aliphatisch-aromatischen oder aromatischen Rest

n eine ganze Zahl von 1 bis 3

m 1 oder 2 und

Q Sauerstoff oder Schwefel bedeutet,

Verfahren zu ihrer Herstellung und ihre Verwendung zur Herstellung hitzebeständiger Beschichtungsmaterialien, Folien, Kleber, Pulver, Formkörper und Lacke.

EP 0 002 445 A1

BAYER AKTIENGESELLSCHAFT 5090 Leverkusen, Bayerwerk
Zentralbereich
Patente, Marken und Lizenzen Str-kl

Heterocyclische Polyisocyanate (III), ihre Herstellung und Verwendung

Die Erfindung betrifft Polyiso(thio)cyanate mit mindestens einer durch Carbonsäureamidgruppen substituierten Hydantoin- oder Thiohydantoinring im Molekül, deren Herstellung durch Umsetzung von Äthylen-1,2-dicarbonsäureanhydriden, Aminoalkoholen und einem Überschuß an Polyiso(thio)cyanaten, sowie deren Verwendung zur Herstellung hitzebeständiger Beschichtungsmittel.

Verfahren zur Herstellung von Hydantoinen (J. Am. Che. 45/383) bzw. Polyhydantoinen (Be 678 282) sind bekannt. Auch die Hydantoinherstellung durch Umsatz von Äthylen-1,2-dicarbonsäureestern, Aminen und Isocyanaten ist beschrieben worden. Dabei werden aber in der Regel äquivalente Mengen der Reaktionspartner eingesetzt, da man bei Verwendung eines Überschusses eines Reaktionspartners, insbesondere des Polyisocyanats befürchten mußte, daß die Polyisocyanate in Gegenwart der Amine bevorzugt zu Polyisocyanuraten und nicht unter Ringschluß zu den gewünschten Hydantoinen reagieren.

Überraschenderweise wurde nun gefunden, daß trotz eines Überschusses an Polyisocyanten die Herstellung von mit Carbonsäure-

amidgruppen substituierten Hydantoinen reibungslos verläuft und man dabei neue Hydantoingruppen-haltige Polyiso(thio)cyanate erhält, die wertvolle Zwischenprodukte darstellen.

Gegenstand der Erfindung sind daher neue Hydantoingruppenhaltige Poly(thio)isocyanate, vorzugsweise solche der allgemeinen Formel (I)

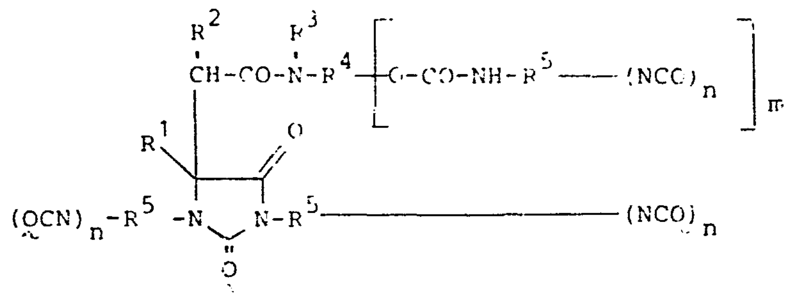

(I)

in der

$R^1$ und $R^2$ gleich oder verschieden, gegebenenfalls substituierte aliphatische, aliphatisch-aromatische oder aromatische Reste, Wasserstoff oder Halogen,

$R^4$ und $R^5$ gleich oder verschieden, einen mindestens zweiwertigen, gegebenenfalls substituierten aliphatischen, aliphatisch-aromatischen oder aromatischen Rest,

$R^3$ Wasserstoff, einen einwertigen, gegebenenfalls substituierten aliphatischen, aliphatisch-aromatischen oder aromatischen Rest

n eine ganze Zahl von 1 bis 3, vorzugsweise 1 bis 2, besonders bevorzugt 1

m eine ganze Zahl von 1 bis 2, vorzugsweise 1

Q Sauerstoff oder S ist.

Ein weiterer Gegenstand der Erfindung ist ein Verfahren zur Herstellung von Polyiso(thio)cyanaten, die mit Carbonsäureamidgruppen substituierte Hydantoinringe enthalten, durch Umsetzung von gegebenenfalls substituierten Äthylen-1,2-dicarbonanhydriden mit primären oder sekundären Aminoalkoholen zu den entsprechenden (isomeren) Halbamiden und anschließende Reaktion mit Polyisc(thio)-cyanaten, das dadurch gekennzeichnet ist, daß das Äquivalenzverhältnis von Iso(thio)cyanatgruppen zu dem Halbamid der Äthylendicarbonsäure mindestens 1,1 beträgt.

Der Reaktionsverlauf kann schematisch durch folgende Formelgleichung wiedergegeben werden.

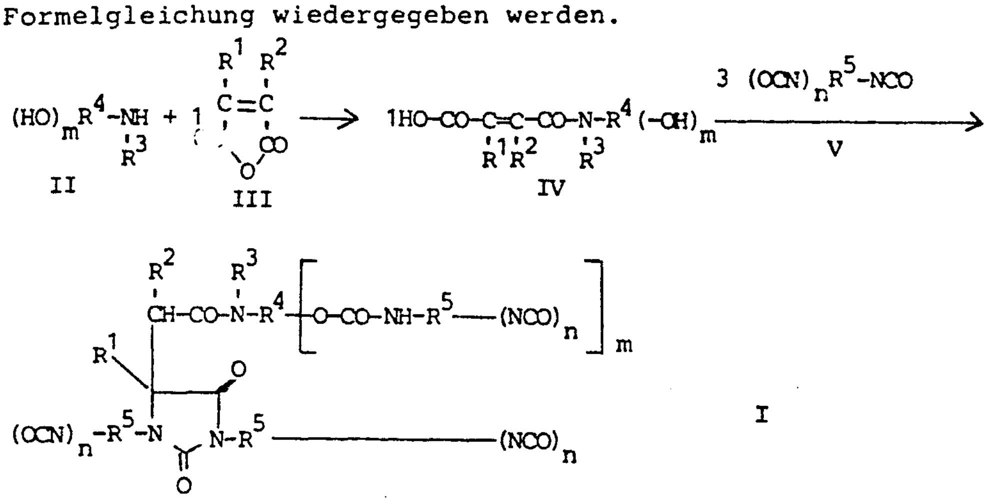


II III IV I

In der Formelgleichung haben $R^1$, $R^2$, $R^3$, $R^5$, x und m die oben angegebene Bedeutung.

Zur Herstellung der erfindungsgemäß neuen Polyiso(thio)cyanate werden OH-gruppenhaltige Halbamide $\alpha$,ß-ungesättigter Dicarbonsäuren eingesetzt, die in situ oder in einer vorgeschalteten Reaktion aus $\alpha$,ß-ungesättigten 1,2-Dicarbonsäureanhydriden und primären oder sekundären Aminoalkoholen in bekannter Weise hergestellt werden.

Neben dem Maleinsäureanhydrid können auch vorzugsweise Halogen oder $C_1$-$C_6$ alkylsubstituierte Äthylen-1,2-dicarbonsäureanhydride, vorzugsweise Methyl-, Dimethyl-, Äthyl-, Butyl-, oder $\Delta^1$-Cyclohexan-1,2-dicarbonsäureanhydride zur Reaktion mit den Aminoalkoholen gebracht werden. $R^1$ und $R^2$ in den oben angegebenen allgemeinen Formeln sind daher vorzugsweise, gleich oder verschieden, Wasserstoff, Halogen wie Chlor oder Fluor, ein Alkylrest mit $C_1$-$C_6$ oder ein Cyclcalkylrest mit $C_5$-$C_8$, ein Alkylarylrest mit $C_7$-$C_{16}$, ein Arylrest mit $C_6$-$C_{20}$ und können zusammen auch einen Ring mit bis zu acht Ringgliedern bilden. Besonders bevorzugt verwendet wird das Anhydrid der Maleinsäure.
Die zum Aufbau der OH-gruppensubstituierten Halbamide eingesetzten Hydroxyaminoverbindungen können gegebenenfalls substituierte aliphatische, cycloaliphatische, araliphatische, aromatische oder auch heterocyclische Verbindungen sein, die neben mindestens einer Hydroxylgruppe eine primäre oder sekundäre Aminogruppe im Molekül enthalten. $R_3$ hat die oben bereits für $R_1$ und $R_2$ angegebene Bedeutung, kann aber für $C \geq 2$ auch mit OH-Gruppen substituiert sein. Vorzugsweise bedeutet $R^4$ in der allgemeinen Formel II der Aminohydroxyverbindung einen aliphatischen Rest mit $C_2$-$C_{10}$, einen cycloaliphatischen Rest mit $C_5$-$C_{10}$, einen araliphatischen Rest mit $C_7$-$C_{16}$, einen aromatischen Rest mit $C_6$-$C_{20}$ oder einen Heteroatom wie N, O oder S enthaltenden Aryl- oder Cycloalkylrest mit $C_5$-$C_{12}$. Besonders bevorzugt steht $R^4$ für einen Alkylrest mit $C_2$-$C_4$, einen Cycloalkylrest mit $C_5$-$C_{10}$ oder einen Arylrest mit $C_6$-$C_{10}$.

Als besonders bevorzugt verwendete Vertreter seien Äthanolamin, Propanolamin, Butanolamin, Aminomethylphenyl, Aminophenol, deren N-Methyl-, N -Äthyl- oder N-Butylderivate, Di-

äthanolamin, Dipropanolamin, 1-Amino-2,3-propandiol oder 1-Amino-1,3-propandiol genannt.

Die Addition der Hydroxyaminoverbindungen an die Äthylen-1,2-dicarbonsäureanhydride erfolgt im allgemeinen bei Temperaturen von -20°C bis 150°C, bevorzugt bei 0°C bis 100°C.

Gegebenenfalls können bei der Addition Katalysatoren wie Kaliumcarbonat, Trialkylamin, Endoäthylenpiperazin, Essigsäure mitverwendet werden. Bei weniger löslichen bzw. höher schmelzenden Komponenten wird die Reaktion im allgemeinen zweckmäßigerweise in organischen Lösungsmitteln durchgeführt, wie sie auch für die Folgereaktionen verwendet werden können und anschließend beschrieben sind. Die Mengenverhältnisse zwischen $\alpha,\beta$-ungesättigten Carbonsäureanhydriden und Aminoalkoholen sollten zweckmäßig stöchiometrisch gewählt werden. Überschüssige Anteile der einen oder anderen Komponente können jedoch eingesetzt und durch an sich bekannte, erfindungsgemäß nicht erfaßte Folgereaktionen in die Polymeren eingebaut werden.

Anstelle der in situ hergestellten Hydroxylgruppen-haltigen Halbamide können natürlich auch auf beliebigen Wegen hergestellte, gegebenenfalls gereinigte Vertreter dieser Substanzklasse in jeder isomeren Form eingesetzt werden.

Gegebenenfalls kann die Cis/trans-Isomerisierung durch Wärme, Basen und/oder Halogene unterstützt bzw. erzielt werden.

Als erfindungsgemäß einzusetzende Polyisocyanate kommen aliphatische, cycloaliphatische, araliphatische, aromatische und heterocyclische Polyisocyanate in Betracht (vgl. Annalen 562, Seiten 75 bis 136), beispielsweise Äthylen-diisocyanat, 1,4-Tetramethylendiisocyanat, 1,6-Hexamethylendiisocyanat, 1,12-Dodecandiisocyant, Cyclobutan-1,3-diisocyanat, Cyclohexan-1,3- und -1,4-diisocanant sowie beliebige Gemische dieser Isomeren, 1-Isocyanato-3,3,5-trimethyl-5-isocyanato-methyl-cyclohexan (DAS 1 202 785), 2,4- und 2,6-Hexahydrotoluylendiisocyanat sowie beliebige Gemische dieser Isomeren, Hexahydro-1,3- und/oder -1,4-phenylen-diisocyanat, Perhydro-2,4'- und/oder -4,4'-diphenylmethan-diisocyanat, 1,3- und 1,4-Phenylendiisocyanat, 2,4- und 2,6-Toluylendiisocyanat sowie beliebige Gemische dieser Isomeren, Diphenylmethan-2,4'- und/oder -4,4'-diisocyanat, Naphthylen-1,5-diisocyanat, Triphenylmethan-2,2',4"-triisocyanat, Polyphenylpolymethylen-polyisocyanate, wie sie durch Anilin-Formaldehyd-Kondensation und anschließende Phosgenierung erhalten und z.B. in den britischen Patentschriften 874 430 und 848 671 beschrieben werden, perchlorierte Arylpolyisocyanate, wie sie z.B. in der deutschen Auslegeschrift 1 157 601 beschrieben werden, Carbodiimidgruppen aufweisende Polyisocyanate, wie sie in der deutschen Patentschrift 1 092 007 beschrieben werden, Diisocyanate, wie sie in der US-Patentschrift 3 492 330 beschrieben werden, Allophanatgruppen aufweisende Polyisocyanate, wie sie z.B. in der britischen Patentschrift 994 890, der belgischen Patentschrift 761 626 und der veröffentlichten holländischen Patentanmeldung 7 102 524 beschrieben werden, Isocyanuratgruppen aufweisende Polyisocyanate, wie sie z.B. in den deutschen Patentschriften 1 022 789, 1 222 067 und 1 027 394 sowie in den deutschen Offenlegungsschriften 1 929 034 und 2 004 048 beschrieben werden, Urethangruppen aufweisende Polyisocyanate, wie sie z.B. in der belgischen Patentschrift 752 261 oder in der US-Patentschrift 3 394 164 beschrieben werden, acylierte Harnstoffgruppen aufweisende Polyisocyanate gemäß der deutschen Patentschrift 1 230 778, Biuretgruppen aufweisende Polyisocyanate, wie sie z.B. in der deutschen Patentschrift

1 101 394, in der britischen Patentschrift 889 050 und in der französischen Patentschrift 7 01 514 beschrieben werden, durch Telomerisationsreaktionen hergestellte Polyisocyanate, wie sie z.B. in der belgischen Patentschrift 723 640 beschrieben werden, Estergruppen aufweisende Polyisocyanate, wie sie z.B. in den britischen Patentschriften 956 474 und 1 072 956, in der US-Patentschrift 3 567 763 und in der deutschen Patentschrift 1 231 688 genannt werden, Umsetzungsprodukte der obengenannten Isocyanate mit Acetalen gemäß der deutschen Patentschrift 1 072 358.

Es ist auch möglich, die bei der technischen Isocyanatherstellung anfallenden, Isocyanatgruppen aufweisenden Destillationsrückstände, gegebenenfalls gelöst in einem oder mehreren der vorgenannten Polyisocyanate, einzusetzen. Ferner ist es möglich, beliebige Mischungen der vorgenannten Polyisocyanate zu verwenden.

Vorzugsweise eignen sich Polyisocyanate der allgemeinen Formel

$$R^5(NCO)_{n+1} \qquad (V)$$

in denen $R^5$, wie in der allgemeinen Formel (I), für einen, gegebenenfalls substituierten aliphatischen Rest mit 2 - 20 C-Atomen, einen aromatischen Rest mit 5 - 12 C-Atomen, einen cycloaliphatischen Rest mit 5 - 12 C-Atomen, einen aliphatisch-aromatischen Rest mit 6 - 20 C-Atomen und

einen Heteroatome wie N, O oder S enthaltenden aromatischen oder cycloaliphatischen Rest mit 5 - 12 C-Atomen steht. n ist eine ganze Zahl von 1 -3, vorzugsweise 1 - 2 , besonders bevorzugt 1.

Die Reste können alle durch Halogen, Alkyl- mit $C_1$-$C_6$ und/oder Arylgruppen mit $C_6$-$C_{16}$ substituiert sein.

Bevorzugt verwendet werden die technisch leicht zugänglichen Gemische aus Toluylen-diisocyanaten, m-Phenylendiisocyanat, sowie phosgenierte Kondensate aus Anilin und Formaldehyd mit Polyphenylen-methylen-struktur und die symmetrischen Verbindungen 4,4'-Diisocyanatodiphenylmethan, 4,4'-Diisocyanatodiphenyläther, p-Phenylendiisocyant und 4,4'-Diisocyanatodiphenyldimethylmethan, Isophorondiisocyanat sowie 4,4'-Diisocyanatodiphenylsulfid und Hexamethylendiisocyanat.

Die Isocyanate können in freier Form, ferner zum Teil oder vollständig auch in Form ihrer beim Umsatz mit reaktiven Wasserstoff enthaltenden Verbindungen zugänglichen und unter den Reaktionsbedingungen als Isocyanat-Abspalter reagierenden Derivate eingesetzt werden.

Vorzugsweise werden als Abspalter die aus aromatischen und aliphatischen Mono- und Polyhydroxy-Verbindungen enthaltenden Carbamidsäureester und die Additionsprodukte aus Lactamen, Oximen und CH-aciden Verbindungen eingesetzt.

Als Beispiele seien die Carbamidsäureester aus Phenol, isomeren Kresolen, deren technischen Gemischen und ähnlichen aromatischen Hydroxylverbindungen, aliphatische Monoalkohole wie Methanol, Äthanol, Propanol, Isopropanol, Butanol, Isobutanol, Cyclohexanol, Allylalkohol, Benzylalkohol und aliphatische Di- oder Polyole wie Äthylenglykol und Trimethylolpropan, weiterhin die Additionsprodukte mit Pyrrolidon-(2), Caprolactam, Butanon-oxim, Malonester, Acetessigester und Acetophenon aufgeführt. Ferner kommen hier die Monoäther von Äthylen- und Diäthylenglykol bzw. Propylen- und Dipropylenglykol mit Methanol, Äthanol, Butanol, Benzylalkohol oder Phenolen in Frage.

Die Isocyanat-Abspalter können als solche eingesetzt oder erst in situ durch Umsetzung mit den entsprechenden Reaktanten erzeugt werden.

Anstelle der genannten (Poly)Isocyanate können auch die analogen (Poly)-Isothiocyanate als Ausgangmaterialier benutzt werden.

Die Mengenverhältnisse zwischen den OH-gruppenhaltigen Halbamiden und Polyisocyanaten müssen so gewählt werden, daß die erhaltenen Reaktionsprodukte überschüssige Isocyanatgruppen, möglichst über 10%, vorzugsweise über 30%, gegebenenfalls in verkappter Form, enthalten sollen. Hierzu ist es erforderlich, daß das Äquivalenzverhältnis von Isocyanatgruppen zu den Halbamiden mindestens 1,1 beträgt. Dies bedeutet, daß z.B. pro Mol Halbamid mit einer alkoholischen OH-Gruppe mehr als 3 Äquivalente an Isocyanatgruppen der einzusetzenden Polyisocyanatkomponente verwendet werden müssen, wobei allerdings überschüssiges eingesetztes Polyiso-

cyanat, welches gegebenenfalls anschließend entfernt oder auch als zusätzliches nicht erfindungsgemäßes Polyisocyanat im Reaktionsprodukt belassen werden kann, nicht stört.

Die erfindungsgemäße Umsetzung der Hydroxygruppen-haltigen Halbamide mit den Polyisocyanaten zu den beanspruchten Polyhydantoingruppen enthaltenden Polyisocyanaten erfolgt gegebenenfalls in Lösungsmitteln, die gegenüber den Reaktionskomponenten inert sind oder aber speziell gegenüber der Polyisocyanatkomponente zu Verbindungen mit spaltbaren Additionsverbindungen befähigt sind, z.B. Phenole Lactame, Alkohole, Kohlenwasserstoffe, Halogenkohlenwasserstoffe, Ester, cyclische Ester, Ketone, Äther, substituierte Amide, Nitrile. Genannt seien beispielsweise Phenol, techn. Kresolgemische, ε-Caprolactam, Acetophenon, Äthylenglykolbutyläther, Diäthylenglykolmethyläther, Cyclohexanon, Glykolmonomethylätheracetat, γ-Butyrolacton, ε-Caprolacton, Benzosäurealkylester, N-Methylpyrrolidon, Dimethylformamid, Dimethylacetamid, Benzoenitril und andere sowie deren Gemische.

Zur Umsetzung der Halbamide mit den Polyisocyanaten werden die Reaktionskomponenten, gegebenenfalls mit den Lösungsmitteln einige Minuten bis zu mehreren Stunden bei Temperaturen von -20°C bis 350°C, bevorzugt 0°C bis 250°C, gehalten. Der Reaktionsverlauf kann über die IR-Spektren verfolgt werden.

Die hohe Geschwindigkeit der erfindungsgemäßen Umsetzung gestattet auch deren Durchführung in Gegenwart phenolischer und/oder alkoholischer Hydroxylgruppen und/oder Amidgruppen, d.h. derartige Gruppierungen können in den Umsetzungskomponenten, gegebenenfalls als Blockmittel, durchaus vorhanden sein, bzw. überschüssige Hydroxyl- bzw. Lactam-Komponenten können auch als Lösungsmittel verwendet werden.

Die Durchführung dieser Reaktion kann gegebenenfalls unter Inertgas vorgenommen werden.

Die Acidität saurer Lösungsmittel wie der Phenole bzw. Kresole genügt zur Reaktionsführung innerhalb hinreichend kurzer Reaktionszeiten. In inerten Medien oder in Schmelzansätzen können beispielsweise Carbonsäuren mit hinreichendem Schmelz- bzw. Siedepunkt wie Essigsäure, Benzoesäure, Bernsteinsäure, Benzoldicarbonsäuren, Butantetracarbonsäure, Trimelitsäure bzw. deren Anhydride gegebenenfalls auch als einbaufähige Katalysatoren in Mengen von 0,1 bis 40, vorzugsweise von 1 bis 10 Prozent in Val, bezogen auf ein Val Isocyanat, mitverwendet werden.

Zur weiteren Beschleunigung der ablaufenden Reaktionen sind die aus der Isocyanatchemie geeigneten bekannten Basen, Säuren und/oder Metallkatalysatoren wie Triäthylamin, N-Methylmorpholin, Endoäthylenpiperazin, Titantetrabutylat, Titanaminoalkoholat, Eisenacetylacetonat, Dibutylzinndilaurat, Bleioctoat, Essigsäure oder p-Toluolsulfonsäure zu verwenden.

Wie bereits erwähnt, kann die erfindungsgemäße Umsetzung auch in situ aus den einzelnen Komponenten, d.h. den Aminoalkoholen, den $\alpha,\beta$-ungesättigten 1,2-Dicarbonsäureanhydriden und den Polyiso(thio)cyanaten in einem Reaktionsschritt durchgeführt werden. Eine weitere Variante des Verfahrens besteht in einer dergestalt verlaufenden Reaktionsfolge, daß die aus der Aminoalkoholkomponente und den Polyiso-(thio)-cyanaten erhaltenen primären Additionsprodukte durch Zugabe des $\alpha,\beta$-ungesättigten 1,2-Dicarbonsäureanhydrids in die erfindungsgemäßen Hydantoingruppen enthaltenden Polyisocyanate übergeführt werden, indem sich unter den Reaktionsbedingungen der erfindungsgemäßen Umsetzung offensichtlich Gleichgewichte der entsprechenden Reaktionskomponenten einstellen.

Die erfindungsgemäßen Hydantoingruppen enthaltenden Polyisocyanate stellen hochviskose bis spröde Massen dar, die je nach ihrem Anwendungszweck in konzentrierter Form oder auch als Lösungen in den bereits früher aufgeführten Lösungsmitteln vorliegen können.

Die erfindungsgemäßen Hydantoingruppen enthaltenden Polyisocyanate werden zur Herstellung von Polyurethankunststoffen, vorzugsweise von Elektroisolierstoffen, eingesetzt. Hierbei können die erfindungsgemäßen Polyisocyanate direkt mit den üblichen Polyestern, Polyesterimiden, Polyamidimiden, Polyesteramidimiden, Polyesterhydantoinen unter Ausbildung von Carbamidesterstrukturen nach den bekannten Methoden eingebrannt werden. Eine weitere Variante für die beschriebenen Polyisocyanate besteht darin, daß sie als Polyisocyanatkomponente in den üblichen Verfahren zur Herstellung von gegebenenfalls Estergruppen aufweisenden Polyimiden, Polyamidimiden und Polyhydantoinen eingesetzt werden können. Diese Reaktion kann sowohl in einer vorgeschalteten Operation als auch in situ zusammen mit dem Einbrennvorgang vorgenommen werden. Die hierzu angewendeten Verfahrensweisen gehen beispielsweise aus den Be 678 282, DP 1 035 362 hervor.

**Beispiel 1:**

| | |
|---|---|
| 522,6 g | Toluylendiisocyanat (Isomerengemisch 2,4 : 2,6 wie 80 : 20) werden unter $N_2$ in |
| 100 g | Acetophenon gelöst, ab 20 bis 150°C portionsweise mit der zuvor unter $N_2$ bei 5 bis höchstens 70°C hergestellten und anhydridfreien Mischung aus |
| 98,1 g | Maleinsäureanhydrid, |
| 402 g | Carbitol (Diäthylenglykolmonoäthyläther), |
| 12 g | ε-Caprolactam und |
| 75 g | N-Methylaminoäthanol kombiniert, 1 Stunde bei 70°C nachgerührt, mit |
| 0,5 g | Endoäthylenpiperazin versetzt und über 100/120°C bei 150 bis 160°C in ca. 5 Stunden bis zum Abschluß der zunächst recht heftigen $CO_2$-Entwicklung sowie Verbrauch der NCO-Gruppen gerührt und abschließend noch je 0,5 Stunden bei 175 bzw. 200-210°C getempert. Dann versetzt man bei 120-140°C mit insgesamt |
| 384,2 g | Trimelitsäureanhydrid |
| 166,1 g | Isophthalsäure |
| 388,4 g | Terephthalsäuredimethylester, homogenisiert im Zuge der $CO_2$-Entwicklung durch langsames Anheizen über 150/170°C auf 200°C und kondensiert ca. 3 Stunden bzw. bis zum Ende der $CO_2$-Entwicklung bei 200-210°C, gibt dann bei 120°C-130°C |
| 276,3 g | Glycerin |
| 1,0 g | Butyltitanat hinzu und kondensiert noch mal ca. 5 Stunden bei 200-220°C, zuletzt ca. 1 Stunde unter Vakuum bei 200-210°C. |

Abschließend wird der Ansatz bei 150 - 120°C mit

1470 g Acetophenon verdünnt, mit der Lösung von

16 g Titantetrabutylat in

32 g Acetylaceton versetzt und noch 1 Stunde bei 120 - 100°C homogenisiert.

Die ca. 50 %ige Lacklösung besitzt eine Viskosität von 7300 $cP_{20^\circ C}$.

Der mit diesem Drahtlack in einem 4 m Ofen bei 9 m/Minute beschichtete Cu-Draht, von 0,7 mm besitzt eine Erweichungstemperatur von >330°C, einen Hitzeschock von >240°C und eine Dauerwärmebeständigkeit von ca. 14 Tagen bei 200°C.

Beispiel 2:

44,1 g Maleinsäureanhydrid in

500 g γ-Butyrolacton werden ca. 1 Minute auf 100°C erhitzt, dann bei 20°C unter $N_2$ mit

33,8 g N-Methylaminoäthanol versetzt, ca. 1 Stunde bei 70°C bis zum Verbrauch der Anhydridgruppen nachgerührt, schließlich bei 30°C mit

298,9 g N,N'-Bis-[2-methoxycarbonylpropyl-(2)]-4,4'-diaminodiphenylmethan vermischt und homogenisiert. Diese Lösung versetzt man dann bei 30-45°C mit der Lösung von

500,4 g 4,4'-Diisocyanatodiphenylmethan

200 g Toluol, rührt ca. 3 Stunden bei 20-35°C nach,

homogenisiert bei 50 - 75°C mit

153,7 g Trimelitsäureanhydrid und

132,9 g Isophthalsäure, fügt

0,5 g Endoäthylenpiperazin zu und kondensiert ca. 5 Std. bei 220°C

Anschließend wird bei 120°C mit

194,1 g Terephthalsäuredimethylester und

184,0 g Glycerin sowie

1,0 g Butyltitanat versetzt, über 140 - 150°C ca. 6 Stunden auf 200 - 220°C erhitzt und zuletzt noch ca. 1 Stunde unter Vakuum bei 200 - 210°C nachkondensiert.

Abschließend verdünnt man den Ansatz bei 150-120°C mit

1011 g Benzoesäuremethylester, versetzt mit der Lösung von

13 g Titantetrabutylat in

26 g Acetylaceton und homogenisiert noch ca. 1 Stunde bei 100 - 120°C.

Die ca. 50 %ige Lacklösung besitzt eine Viskosität von 3800 cP 20°. Der in einem 4 m Ofen bei 8 m/min. hergestellte 0,7 mm Cu-Lackdraht besitzt eine Erweichungstemperatur von > 330°C, einen Hitzeschock von > 260°C, eine Dauerwärmebeständigkeit von > 7 Tagen bei 200°C, eine Schabefestigkeit von 109 Doppelhüben, eine Durchschlagfestigkeit von 9 kV sowie eine gute Chemikalienbeständigkeit.

Beispiel 3

1501,2 g 4,4'-Diisocyanotodiphenylmethan werden unter $N_2$ ab 50 - 150°C portionsweise mit der zuvor unter $N_2$ bei 5 bis höchstens 30°C hergestellten Lösung von

196 g Maleinsäureanhydrid in

844 g Carbitol

21 g ε-Caprolactam

150 g ω-Aminopropanol

kombiniert, bei 150°C bis zum Abschluß der zunächst sehr heftigen $CO_2$-Entwicklung nachgerührt und schließlich ca. 0,5 Stunden bei 200 - 220°C unter Rückfluß gerührt.

Das schwarz-braune, homogene Harz mit den typischen Hydantoin-IR-Banden besitzt einen latenten NCO-Gehalt von ca. 9,5 % (theoret.: 13 %) und in 70 %iger Carbitollösung eine Viskosität von ca. 11570 cP 20°C.

Beispiel 4

In die Lsg. von 750 g 4,4'-Diisocyanato-diphenylmethan in 600 g Butyrolacton werden bei 110 - 120°C nach Maßgabe der Wärmeentwicklung 324 g eines technischen Kresolgemisches eingetropft. Dann wird noch 0,5 Stdn. bei 120°C nachgerührt. Anschließend wird auf 0°C abgekühlt und unter Kühlung bei dieser Temperatur 473 g einer 31 %igen Lsg. von N-Hydroxyäthyl-N-methyl-maleinamidsäure in Butyrolacton, die aus N-Methyl-äthanolamin und Maleinsäureanhydrid hergestellt wurde, eingetragen. Danach wird jeweils 1 Stde. bei 10, 20, 30, 50 und 75°C und 6 Stdn. bei 100°C gerührt. Die Kondensation erfolgt unter Abspaltung von Kohlendioxid. Nach Zugabe von 11 g Kresol erhält man das Isocyanato-hydantoin als braune viskose Lsg. mit einer Viskosität von $\eta^{25}$ = 83 000 mPa s. Der Gehalt an verkapptem Isocyanat beträgt 6,5 %.

Aus 100 g dieser Lsg. wird mit 150 g eines Polyesters aus Terephthalsäure, Äthylenglykol und Glycerin, 400 g Kresol und 1 g Titantetrabutylat eine Lacklsg. bereitet, die auf ein Blech aufgestrichen und in jeweils 15 Min. bei 200°C und 300°C zu einem klaren elastischen Lackfilm eingebrannt wird.

Beispiel 5

255 g eines technischen Kresolgemisches, 31 g Äthylenglykol und 105 g der nach Beispiel 4 hergestellten Lsg. eines Isocyanato-hydantoins werden vorgelegt und bei 120°C zuerst mit 70 g eines Gemisches aus 80 Tln. 2,4- und 20 Tln. 2,6-Toluylendiisocyanat und dann mit 192 g Trimellitsäureanhydrid versetzt. Das Gemisch wird zur Kondensation, die unter Abspaltung von Kohlendioxid und Wasser verläuft, jeweils 2 Stdn. bei 170°C, 180°C, 190°C, 200°C und 205°C gerührt. Man erhält das Hydantoin-esterimid als braune viskose Lsg., die mit gleichen Teilen Phenol und Kresol auf einen Festgehalt von 40 % verdünnt wird und deren Viskosität $\eta^{25}$ dann 14 300 mPa s beträgt.

Zur Bereitung einer Lacklsg. werden 250 g eines Polyesters aus Terephthalsäure, Äthylenglykol und Glycerin und 7,5 g Titantetrabutylat als Katalysator zugegeben und mit Kresol auf einen Festgehalt von 30 % verdünnt.

Auf einer Drahtlackiermaschine wird mit dieser Lsg. ein Cu-Draht von 0,7 mm Ø lackiert.

Ofenlänge: 4 m
Ofentemperatur: 400°C
Anzahl der Durchzüge: 6.

Unter diesen Bedingungen wird bei einer Abzugsgeschwindigkeit des Drahtes von 11 m/Min. ein beschichteter Draht mit einer max. Außenfaserdehnung von 88 % und einem Hitzeschock von 260°C erhalten.

Beispiel 6

In die Lsg. von 750 g 4,4'-Diisocyanato-diphenylmethan in 600 g Butyrolacton werden bei 90°C unter Kühlung 222 g n-Butanol eingetropft. Dann wird auf 0°C abgekühlt und anteilweise 473 g einer 31 %igen Lsg. von N-Hydroxyäthyl-N-methyl-maleinamidsäure in Butyrolacton zugegeben. Danach wird zur Ausführung der Kondensation und Cyclisierung jeweils 1 Stde. bei 10, 20, 30 und 50°C und 10 Stdn. bei 100°C gerührt. Nach Beendigung der Reaktion werden noch 7,4 g n-Butanol eingerührt und man erhält eine Lsg. des mit Butanol verkappten Isocyanato-hydantoins, deren Gehalt an freiem Isocyanat < 0,5 % ist.

200 g dieser Lsg. werden mit 100 g eines Polyesters aus Terephthalsäure, Glykol und Glycerin und 1 g Titantetrabutylat versetzt, mit Kresol auf einen Festgehalt von 30 % verdünnt und unter den in Beispiel 2 beschriebenen Bedingungen auf einen Kupferdraht von 0,7 mm ∅ aufgebracht. Man erhält einen Lackdraht mit einer Erweichungstemperatur von 309°C und einer Abriebfestigkeit von 26 Hüben.

Beispiel 7

In die Lsg. von 522 g eines Gemisches aus 80 Tln. 2,4- und 20 Tln. 2,6-Toluylendiisocyanat werden bei 110 - 120°C 324 g eines technischen Kresolgemisches eingetropft. Anschließend wird noch 1 Stde. bei dieser Temperatur gerührt. Dann wird abgekühlt und bei 0°C 473 g einer 31 %igen Lsg. von N-Hydroxyäthyl-N-methyl-maleinamidsäure in Butyrolacton zugetropft. Die Temperatur des Reaktionsgemisches wird unter Rühren innerhalb von 6 Stdn. auf 100°C gesteigert und dann 2 Stdn. bei 100°C, 1 Stde. bei 110°C und 1 Stde. bei 120°C gehalten. Man erhält das mit Kresol verkappte Isocyanato-hydantoin als braune viskose Lsg., die zur Bereitung einer

Lacklsg. mit einem Polyester aus Terephthalsäure, Glykol und Glycerin im Verhältnis 1 : 2 abgemischt, mit 1 % Titantetrabutylat versetzt und mit Kresol auf einen Festgehalt von 30 % verdünnt wird.

Die Lackierung eines Cu-Drahtes von 0,7 mm ∅ mit dieser Lsg. unter den in Beispiel 2 beschriebenen Bedingungen ergibt bei einer Geschwindigkeit von 7 m/Min. einen Lackdraht mit einer Erweichungstemperatur von 318°C.

Beispiel 8

255 g Butyrolacton, 31 g Äthylenglykol und 130 g eines entsprechend Beispiel 4 hergestellten Isocyanato-hydantoins werden vorgelegt und bei 120°C anteilweise mit 70 g eines Gemisches aus 80 Tln. 2,4- und 20 Tln. 2,6-Toluylendiisocyanat versetzt. Dann werden 192 g Trimellitsäureanhydrid zugegeben und die Kondensation unter Rühren in jeweils 2 Stdn. bei 170, 180, 190, 200 und 205°C ausgeführt. Das Hydantoin-esterimid wird als braune viskose Lsg. erhalten. Die Viskosität $\eta^{25}$ einer mit Kresol auf einen Festgehalt von 40 % verdünnten Lsg. beträgt 2340 mPa s.

Zur Bereitung einer Lacklsg. werden 200 g des Reaktionsproduktes mit 100 g eines Polyesters aus Terephthalsäure, Glykol und Glycerin und 3 g Titantetrabutylat versetzt und mit gleichen Teilen Phenol und Kresol auf einen Festgehalt von 30 % verdünnt. Diese Lacklsg. wird, wie in Beispiel 2 beschrieben, auf einen Kupferdraht von 0,7 mm ∅ aufgebracht. Bei einer Abzugsgeschwindigkeit des Drahtes von 9 m/Min. wird ein Lackdraht erhalten, für den eine maximale Außenfaserdehnung von 88 % und ein Hitzeschock von 260°C gemessen werden.

Beispiel 9

In die Lsg. von 522 g 2,4-Toluylendiisocyanat in 675 g Butyrolacton werden bei 110°C 222 g n-Butanol unter Kühlung eingetropft. Dann wird auf 0°C abgekühlt und 473 g einer 31 %igen Lsg. von N-Hydroxyäthyl-N-methyl-maleinamidsäure eingetragen. Danach wird die Temperatur unter Rühren jeweils pro Stde. um 10°C bis auf 100°C gesteigert und dann noch 2 Stdn. bei 100°C und 2 Stdn. bei 120°C gehalten. Man erhält das mit Butanol verkappte Isocyanato-hydantoin als braune viskose Lsg., deren Gehalt an freiem Isocyanat $<$ 0,4 % ist.

Eine Probe wird im Verhältnis 1 : 1 mit einem Polyester aus Terephthalsäure, Glykol und Glycerin und mit 1,5 % Titantetrabutylat versetzt und mit Kresol auf einen Festgehalt von 30 % verdünnt. Die Lacklsg. wird auf ein Probeblech aufgestrichen und ergibt nach dem Einbrennen in jeweils 15 Min. bei 200 und 300°C einen klaren elastischen Lackfilm.

Patentansprüche

1. (Thio)hydantoingruppenhaltige Polyiso(thio)cyanate der allgemeinen Formel (I)

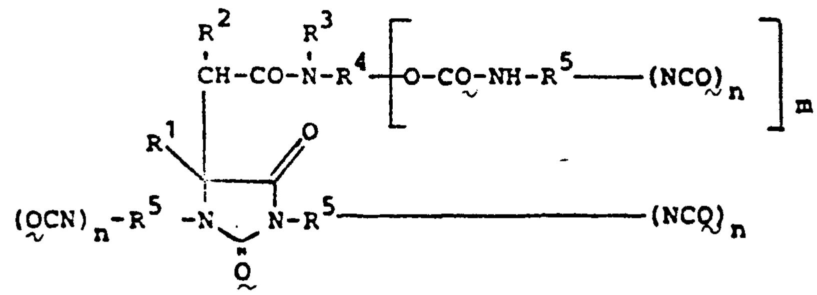

(I)

in der

| | |
|---|---|
| $R^1$ und $R^2$ | gleich oder verschieden, gegebenenfalls substituierte aliphatische, aliphatisch-aromatische oder aromatische Reste, Wasserstoff oder Halogen, |
| $R^4$ und $R^5$ | gleich oder verschieden, einen mindestens zweiwertigen, gegebenenfalls substituierten aliphatischen, aliphatisch-aromatischen oder aromatischen Rest, |
| $R^3$ | Wasserstoff, einen einwertigen, gegebenenfalls substituierten aliphatischen, aliphatisch-aromatischen oder aromatischen Rest |
| n | eine ganze Zahl von 1 bis 3 |
| m | 1 oder 2 und |
| Q | Sauerstoff oder Schwefel bedeutet. |

2. Poly(thio)isocyanate nach Anspruch 1, dadurch gekennzeichnet, daß in der allgemeinen Formel (I)

$R^1$ und $R^2$ gleich oder verschieden, Wasserstoff, Halogen, einen Alkylrest mit $C_1$-$C_6$ oder einen Cycloalkylrest mit $C_5$-$C_8$, einen Alkylarylrest mit $C_7$-$C_{16}$, einen Arylrest mit $C_6$-$C_{20}$ oder zusammen einen Ring mit bis zu acht Ringgliedern bilden,

$R^3$ die Bedeutung von $R^1$ bzw. $R^2$ hat und Reste mit wenigstens 2 C-Atomen mit OH substituiert sein können,

$R^4$ einen aliphatischen Rest mit $C_2$-$C_{10}$, einen cycloaliphatischen Rest mit $C_5$-$C_{10}$, einen araliphatischen Rest mit $C_7$-$C_{16}$, einen aromatischen Rest mit $C_6$-$C_{20}$ oder einen Heteroatom wie N, O oder S enthaltenden Aryl- oder Cycloalkylrest mit $C_5$-$C_{12}$ und

$R^5$ einen aliphatischen Rest mit 2 - 20 C-Atomen, einen aromatischen Rest mit 5 - 12 C-Atomen, einen cycloaliphatischen Rest mit 5 - 12 C-Atomen, einen aliphatisch-aromatischen Rest mit 6 - 20 C-Atomen und einen Heteroatome wie N, O oder S enthaltenden aromatischen oder cycloaliphatischen Rest mit 5 - 12 C-Atomen, die alle durch Halogen, Alkyl- mit $C_1$-$C_6$ und/oder Arylgruppen mit $C_6$-$C_{16}$ substituiert sein können,

bedeuten.

3. Ein Verfahren zur Herstellung von Polyiso(thio)cyanaten, die mit Carbonsäureamidgruppen substituierte (Thio)Hydantoinringe enthalten, durch Umsetzung von gegebenenfalls substituierten Äthylen-1,2-dicarbonanhydriden mit primären oder sekundären Aminoalkoholen zu den entsprechenden (isomeren) Halbamiden und gleichzeitiger oder anschließende Reaktion mit Polyiso(thio)-cyanaten, dadurch gekennzeichnet, daß das Äquivalenzverhältnis von Iso(thio)cyanatgruppen zu dem Halbamid der Äthylendicarbonsäure mindestens 1,1 beträgt.

4. Verfahren nach Anspruch 3, dadurch gekennzeichnet, daß die Umsetzung zunächst bei Temperaturen von -20°C bis 150°C und dann bei -20°C bis 350°C erfolgt.

5. Polyiso(thio)cyanate erhältlich nach Verfahren gemäß Ansprüchen 3 und 4.

6. Verwendung der Polyiso(thio)cyanate gemäß Ansprüchen 1 und 2 zur Herstellung von hitzebeständigen Beschichtungsmaterialien, Folien, Kleber, Pulver, Formkörper und Lacke.

0002445

Europäisches Patentamt

# EUROPÄISCHER RECHERCHENBERICHT

Nummer der Anmeldung

EP 78 101 271.1

| Kategorie | EINSCHLÄGIGE DOKUMENTE<br>Kennzeichnung des Dokuments mit Angabe, soweit erforderlich, der maßgeblichen Teile | betrifft Anspruch |
|---|---|---|
| - | DE - A - 1 670 970 (BAYER)<br>* Ansprüche 1 bis 4, Seite 11 *<br>-- | 1,2,5, 6 |
| - | DE - A - 2 014 859 (CIBA)<br>* Ansprüche 3, 12 *<br>-- | 5,6 |
| A | DE - A - 2 018 043 (CIBA)<br>* ganzes Dokument *<br>-- | |
| A | DE - A - 2 404 741 (BAYER)<br>* ganzes Dokument *<br>-- | |
| - | FR - A - 2 225 426 (BAYER)<br>* Ansprüche 1, 7 *<br>---- | 1,2, 5,6 |

KLASSIFIKATION DER ANMELDUNG (Int.Cl.²)

C 07 D 233/76
C 08 G 18/78
C 09 D 3/49
C 09 J 3/16

RECHERCHIERTE SACHGEBIETE (Int. Cl.²)

C 07 D 233/76
C 08 G 18/78
C 09 D 3/49

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Berlin | 27-02-1979 | FROELICH |

EPA form 1503.1 06.78